# EUROPEAN PATENT APPLICATION

(11) **EP 4 796 149 A1**
(43) Date of publication of application: **26.08.2026**
(21) Application number: 25159238.2
(22) Date of filing: 20.02.2025
(51) Int. Cl.: A61M 11/00, A61M 15/00, A61M 15/08, A61M 16/00, B05B 17/00

(54) **NEBULISER**

(71) Applicant: 3C Project Technologies Limited, Greystones 281603 (IE)
(72) Inventor: MCAVOY, Gregory John, Greystones, 281603 (IE)
(74) Representative: Hindles Limited

(57) **Abstract**

The present invention provides a nebuliser (100) comprising one or more inputs (110), and a plurality of droplet ejectors (126) in fluid communication with the one or more inputs (110). Each droplet ejector comprises a nozzle portion and a piezoelectric actuator. The one or more inputs are for receiving one or more liquids. Each nozzle portion defines a nozzle outlet in fluid communication with the one or more inputs. Each piezoelectric actuator is operable to cause ejection of a liquid received by one of the one or more inputs through the respective nozzle outlet as one or more droplets. The nebuliser is configured to generate an aerosol comprising the respective one or more droplets ejected by one or more of the plurality of droplet ejectors. At least 50%, by mass, of the droplets of the aerosol have a diameter of at least 5 x 10⁻⁶ m.

## Description

### Field of the invention

The present invention relates to a nebuliser for delivering a substance to the olfactory region.

### Background to the invention

Medication is delivered to the human body in a relatively small number of different ways. Most medication is currently delivered orally through pills, or via injection using a needle. Administering medication to the brain, for example to treat neurological disorders, is more challenging. This is due to the presence of the blood brain barrier, which restricts the passage of drugs to the brain via the bloodstream. The olfactory nerve, located in the olfactory region of the nose, provides a direct pathway through the blood brain barrier to the brain and central nervous system. Liquid formulations are known to be effective for delivery of medication in this way. However, the size of the droplets of the administered liquid must be tightly controlled to ensure the substance can be absorbed and transmitted to the brain without unwanted transmission of the droplets to the lungs.

It is in this context that the present invention has been devised.

### Summary of the invention

In accordance with an aspect of the present invention, there is provided a nebuliser for delivering a substance to the olfactory region. The nebuliser comprises one or more inputs for receiving one or more liquids. At least one of the one or more liquids comprises the substance. The nebuliser further comprises a plurality of droplet ejectors together in fluid communication with the one or more inputs. Each droplet ejector comprises a piezoelectric actuator and a nozzle portion defining a nozzle outlet in fluid communication with a one of the one or more inputs. Each piezoelectric actuator is operable to cause ejection of a liquid received by the one of the one or more inputs through the respective nozzle outlet as one or more droplets. The nebuliser is configured to generate an aerosol comprising the respective one or more droplets ejected by one or more of the plurality of droplet ejectors. At least 50%, by mass, (optionally by total number) of the droplets of the aerosol have a diameter of at least 5 × 10⁻⁶ m.

Thus, an aerosol comprising droplets of a liquid comprising a substance to be delivered to the olfactory region can be generated. By directing this aerosol into the nose, the droplets comprising the substance can be delivered to the olfactory region., where they can be transmitted to the brain via the olfactory nerve. By generating an aerosol with droplets having a diameter of at least 5 × 10⁻⁶ m, unwanted transmission of the substance to the lungs is reduced, as heavier droplets are less easily influenced by airflow during inhalation than lighter droplets.

It will be understood that delivering a substance to the olfactory region is delivering a substance such that it is deposited in the olfactory region. In particular, a substance deposited in the olfactory region will not continue to travel through the respiratory system such that it is deposited elsewhere, for example in the lungs. The substance may make contact with the olfactory epithelium. The substance may permeate the olfactory epithelium.

The nebuliser may be for delivering a substance to the brain via the olfactory region. The nebuliser may be configured to deliver a substance to the brain via the olfactory region. The substance may be delivered to the brain via the olfactory nerve. It will be understood that the nebuliser will still be considered to deliver a substance to the olfactory region and/or to the brain even when the substance delivered is a reaction product from an intermediate comprised in the liquid ejected by the droplet ejectors.

In other words, a liquid should still be considered to comprise the substance where it comprises an intermediary for forming the substance.

At least 50% of the droplets of the aerosol may have a diameter of at least 8 × 10⁻⁶ m (8 microns), optionally at least 9 × 10⁻⁶ m (9 microns). Thus, an aerosol comprised of larger, heavier droplets may be generated. These droplets are even more resistant to movement caused by airflow, and so unwanted transmission to the lungs is further reduced.

At least 50% of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns). Thus, the aerosol is more easily able to reach the olfactory region of the nose, bypassing filtration methods such as vibrissae, turbulence, and mucosal contact, than would be possible for an aerosol comprised of larger droplets.

At least 80% of the droplets of the aerosol may have a diameter of at least 5 × 10⁻⁶ m (5 microns). At least 80% of the droplets of the aerosol may have a diameter of at least 8 × 10⁻⁶ m (8 microns), optionally at least 9 × 10⁻⁶ m (9 microns). At least 80% of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns).

At least 90% of the droplets of the aerosol may have a diameter of at least 5 × 10⁻⁶ m (5 microns). At least 90% of the droplets of the aerosol may have a diameter of at least 8 × 10⁻⁶ m (8 microns), optionally at least 9 × 10⁻⁶ m (9 microns). At least 90% of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns).

At least 95% of the droplets of the aerosol may have a diameter of at least 5 × 10⁻⁶ m (5 microns). At least 95% of the droplets of the aerosol may have a diameter of at least 8 × 10⁻⁶ m (8 microns), optionally at least 9 × 10⁻⁶ m (9 microns). At least 95% of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns).

At least 99% of the droplets of the aerosol may have a diameter of at least 5 × 10⁻⁶ m (5 microns). At least 99% of the droplets of the aerosol may have a diameter of at least 8 × 10⁻⁶ m (8 microns), optionally at least 9 × 10⁻⁶ m (9 microns). At least 99% of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns).

Each of the droplets of the aerosol may have a diameter of at least 5 × 10⁻⁶ m (5 microns). Each of the droplets of the aerosol may have a diameter of at least 8 × 10⁻⁶ m (8 microns), optionally at least 9 × 10⁻⁶ m (9 microns). Each of the droplets of the aerosol may have a diameter of no greater than 15 × 10⁻⁶ m (15 microns), for example less than 12 × 10⁻⁶ m (12 microns), optionally no greater than 11 × 10⁻⁶ m (11 microns).

Thus, by providing a nebuliser capable of producing an aerosol comprised of consistently sized droplets of this size, a user is able to generate an aerosol which is able to reach the olfactory region and remain there, without further travel to the lungs.

By providing a plurality of droplet ejectors, each capable of ejecting a plurality of droplets, instead of a single droplet ejector as in vibrating mesh nebulisers, undesired inhomogeneities in the ejection of droplets from different regions of the nebuliser can be reduced, or even completely eliminated. Furthermore, it is possible to provide separate control signals to each of the piezoelectric actuators, allowing for a more finely controlled set of droplets to be ejected through the nozzle outlets. Accordingly, a larger proportion of the droplets present in the aerosol can be controlled to have sizes which correspond to optimum travel to the olfactory region of the nose and deposition and absorption into the olfactory region and then onwards to the brain via the olfactory nerve.

The term nebuliser will be understood to mean substantially any device for converting a liquid being or comprising a medication into an aerosol. The nebuliser may be for use in a domestic or clinical setting, by trained medical professionals, or others, including by the patient themselves. In some examples, it may be that the nebuliser is to be attached to one or more further components to be used. Specifically, any device that includes the plurality of droplet ejectors and one or more inputs described herein can typically be considered a nebuliser. Put another way, it may be that the nebuliser can be considered a refill for a nebuliser apparatus that includes the one or more further components to provide a working device which can be used by a user to generate an aerosol.

The term aerosol as used herein will be understood to mean substantially any droplets of liquid suspended in a gas. Typically, these droplets of liquids will be sufficiently small that they can be inhaled through the nose.

It will be understood that references to a liquid are intended to also include solid or semisolid particulates suspended in liquid in some examples.

The substance may comprise a drug, such as a medication. The substance may be for transporting to the brain via the olfactory region. The substance may be for absorption through the olfactory epithelium. Thus, the substance is able to bypass the blood brain barrier via the olfactory nerve so as to be delivered to the brain. This allows for more effective treatment of, for example, neurological disorders, through brain drug delivery than traditional intravenous or oral administration methods.

The substance may be a liquid. The substance may be a solid. The substance may be a semisolid gel. The substance may be a solid suspended in a one of the one or more liquids. The substance may be soluble in a one of the one or more liquids. The substance may be dissolved in a one of the one or more liquids. The substance may be a gas.

The substance may be or comprise proteins. The substance may be or comprise peptides. The substance may comprise insulin.

At least one of the one or more liquids may comprise a further substance. The further substance may be a priming substance, for example for priming the olfactory region prior to delivery of the substance thereto. The further substance may be for increasing the absorption of the substance into the olfactory epithelium.

The further substance may be a liquid. The further substance may be a solid. The further substance may be or comprise a semisolid gel. The further substance may be a solid suspended in a one of the one or more liquids. The further substance may be soluble in a one of the one or more liquids. The further substance may be dissolved in a one of the one or more liquids.

The further substance may be or comprise a permeation enhancer. For example, the further substance may comprise one or more of the following: cyclodextrins, surfactants, saponins, fusidic acids, phospholipids, bile salts, laureth-9-sulfate, and fatty acids. The further substance may comprise peptides. The further substance may comprise cell-penetrating peptides. The further substance may comprise penetrin. The further substance may comprise bioadhesive materials. The further substance may comprise carbomers. The further substance may comprise polyacrylic acid. The further substance may comprise starch microspheres. The further substance may comprise mucoadhesive agents, for example chitosan.

The further substance may be or comprise enzyme inhibitors, for example peptidase inhibitors. For example, the further substance may be or comprise one or more of the following: bestatin, amastatin, boroleucine, fusidic acids, phospholipids. Thus, metabolism in the nose may be reduced and residence time increased, thereby increasing absorption and improving bioavailability, especially in configurations wherein the substance comprises proteins and/or peptides.

The further substance may be or comprise an absorption enhancer. The further substance may be or comprise trimethyl chitosan complexes. The further substance may be or comprise tamarind seed polysaccharide.

The further substance may be for increasing membrane fluidity. The further substance may be for increasing tight junction permeability. The further substance may be for hydrophilic pore generation. The further substance may be for reducing viscosity. The further substance may be for reducing enzymatic activity.

In some configurations, a one of the one or more liquids may comprise the substance and the further substance.

Typically, the one or more liquids may comprise a first liquid and a second liquid. The one or more liquids may be at least two liquids, such as more than two liquids.

The first liquid may comprise the further substance. The second liquid may comprise the substance.

The one or more inputs typically receive a single liquid at a single respective input. However, in some examples, it may be that a first liquid is received at an input at a first time and a second liquid is received at the input at a second time, subsequent to the first time.

It will be understood that, in some examples, ejection of the one or more droplets through the nozzle outlets need not require that the droplets have sufficient momentum imparted thereto during ejection so as to travel a significant distance from the nebuliser, only that the ejection of the droplets result in formation of the aerosol.

The plurality of droplet ejectors may comprise a first plurality of droplet ejectors and a second plurality of droplet ejectors.

The one or more inputs may comprise a first input and a second input. The first plurality of droplet ejectors may be in fluid communication with the first input. The second plurality of droplet ejectors may be in fluid communication with the second input.

The nebuliser may be configured to generate a first aerosol comprising the respective one or more droplets ejected by the first plurality of droplet ejectors. The nebuliser may be configured to generate a second aerosol comprising the respective one or more droplets ejected by the second plurality of droplet ejectors.

The first input may be for receiving the first liquid. The second input may be for receiving the second liquid.

The first aerosol may be an aerosol of the first liquid. The second aerosol may be an aerosol of the second liquid.

The nebuliser may be configured to commence generation of the first aerosol at a first start time. The nebuliser may be configured to cease generation of the first aerosol at a first end time. The first end time may be after the first start time. The nebuliser may be configured to commence generation of the second aerosol at a second start time. The nebuliser may be configured to cease generation of the second aerosol at a second end time. The second end time may be after the second start time.

The second start time may be the same as the first start time. The second start time may be no earlier than the first start time. The second start time may be after the first start time. The second start time may be after the first end time.

The second start time may be before the first end time. Thus, a first aerosol of a first liquid may be generated, and a second aerosol of a second liquid may be generated while the first aerosol is still being generated, creating a mixture of the two aerosols.

The second start time may be the same as the first end time. The second end time may be before the first end time. The second end time may be the same as the first end time. The second end time may be after the first end time. Thus, two aerosols may be generated at two separate times from the same device. The two aerosols may be of two different liquids.

As such, the nebuliser of the present invention is able to first generate an aerosol comprising a priming substance to increase the uptake of a substance to be delivered to the brain. This increases the range of possible substances which can be delivered to the brain in this manner, without the need for separate devices or more complicated systems.

The nebuliser may be configured to cause a first plurality of droplet ejectors of the plurality of droplet ejectors to generate a first aerosol. The nebuliser may be configured to cause a second plurality of droplet ejectors of the plurality of droplet ejectors to generate a second aerosol.

Each of the droplet ejectors of the first plurality of droplet ejectors may be a different droplet ejector to each of the droplet ejectors of the second plurality of droplet ejectors.

In some embodiments, the first plurality of droplet ejectors may comprise the droplet ejectors of the second plurality of droplet ejectors. That is, the first and second plurality of droplet ejectors are the same plurality of droplet ejectors.

The nebuliser may be configured to commence generation of the first aerosol at a first start time. The nebuliser may be configured to cease generation of the first aerosol at a first end time after the first start time. The nebuliser may be configured to commence generation of the second aerosol at a second start time. The nebuliser may be configured to cease generation of the second aerosol at a second end time after the second start time.

The nozzle outlet defined by at least 50 per cent of the plurality of droplet ejectors may be among no more than one nozzle outlet defined by the respective droplet ejector. The nozzle outlet defined by each of the plurality of droplet ejectors may be among no more than one nozzle outlet defined by the respective droplet ejector. Thus, particularly effective control of the droplet size can be exercised since each piezoelectric actuator, when operated, causes ejection of droplets through only a single nozzle outlet. The nozzle outlet defined by each of the plurality of droplet ejectors may be in fluid communication with the one of the one or more inputs.

The nebuliser may further comprise a storage portion defining one or more cavities for storing the one or more liquids, wherein the one or more cavities are together in fluid communication with each nozzle outlet and with the one or more inputs. Thus, the liquids can be stored in the one or more cavities before ejection by the droplet ejectors.

The storage portion may be removable. The storage portion may be replaceable. Thus, the liquids can be refilled easily by replacing an empty storage portion with a new, full, storage portion. Furthermore, the same nebuliser can be used with multiple different liquids by replacing a first storage portion filled with a first liquid with a second storage portion filled with a second liquid. The one or more cavities may each be individually removable. The one or more cavities may each be individually replaceable. In other examples, the storage portion may be refillable. Thus, the nebuliser can be re-used by refilling the storage portion. It will be appreciated that in many situations it is preferable to replace a storage portion instead of refilling the storage portion *in situ,* so as to ensure a protective environment is maintained (i.e. the liquids are not contaminated).

The nebuliser may further comprise a housing to support the plurality of droplet ejectors. Typically, the housing provides an outer casing of the nebuliser. Thus, the plurality of droplet ejectors may be considered to be in fluid communication with an external environment of the nebuliser. The housing typically provides protection of internal components of the nebuliser from an external environment, as well as providing a structural support on which components of the nebuliser can be mounted.

The storage portion may be part of the housing. Thus, the storage portion can be held securely relative to the one or more inputs, thereby more easily maintaining fluid communication between the one or more cavities and the one or more inputs.

The nebuliser may further comprise an outlet portion defining a nebuliser outlet through which the aerosol can be output from the nebuliser. Thus, the generated aerosol is output from the nebuliser, as is typical in nebulisers.

The outlet portion may be part of the housing. Thus, the aerosol can be conveniently output from the nebuliser using a portion defined in the existing housing of the nebuliser, without requiring a further, separate component.

The housing may comprise a protruding portion. The plurality of droplet ejectors may be provided within the protruding portion. It may be that the plurality of droplet ejectors are provided at an end of the protruding portion, such as an end distal from the rest of the housing. Thus, with the protruding portion inserted into the nose, the aerosol can be generated within the nose and closer to the olfactory region, increasing the proportion of the aerosol deposited at the olfactory region. The protruding portion may be, or may comprise, the outlet portion. The protruding portion may have a diameter such that it may be inserted into the nose.

The outlet portion may define a nebuliser outlet channel to direct the aerosol to be output therefrom in a first direction out of the nebuliser. The outlet portion may be arranged to provide the aerosol internally in the body, such as into the nose. The outlet portion may be in the form of a protrusion, such as a nose insert, to allow for insertion thereof into a nose of a user, for example into a nostril of a user. Thus, the aerosol is able to be output from the nebuliser into the nose of a user. By providing a protruding outlet portion able to be inserted into a nostril of a user, the aerosol is able to bypass hairs present in the outer portion of the nostril which would otherwise further oppose transport of the aerosol to the olfactory region. It will be understood that the nebuliser outlet channel may be provided in the nose insert. It may be that the outlet portion is removeable or replaceable for ease of cleaning, and/or for replacement for different patients/uses.

The nebuliser may further comprise a power supply circuit portion. The power supply circuit portion is arranged to supply power to the piezoelectric actuator of each of the plurality of droplet ejectors. The power supply circuit portion may comprise a battery compartment arranged to receive a battery or a power supply port for connection of a power source thereto. The power supply circuit portion may comprise the power source, such as the battery. The battery may be a rechargeable battery. The nebuliser may further comprise a power source to supply power to the power supply circuit portion. Thus, the nebuliser can be easily moved to different locations. In some examples, the nebuliser may be man-portable, such as hand-portable. The nebuliser is typically handheld.

The power supply circuit portion may be supported by the housing. It may be that the power supply circuit portion is contained within the housing. Thus, protection of the power supply circuit portion can be at least partially provided by the housing.

The nebuliser may further comprise a switch to activate the plurality of droplet ejectors. Thus, the plurality of droplet ejectors need only be activated when required. The switch may be user-operable, or may be operated in dependence on control logic from a controller.

The switch may be a flow switch responsive to a flow through the nebuliser outlet. Thus, it can be considered that the plurality of droplet ejectors may be configured to activate in response to inhalation by the user. The plurality of droplet ejectors may be configured to deactivate in response to cessation of inhalation by the user. In this way, wastage of the liquid to be nebulised can be reduced or even entirely eliminated, as well as making it easier to determine the dosage of the liquid having been delivered to the user.

It may be that, in a first configuration, the switch is in a first state to cause activation of the piezoelectric actuators of the droplet ejectors to thereby cause aerosol to be generated, and in a second configuration, the switch is in a second state corresponding to no aerosol being generated by activation of the piezoelectric actuators. The nebuliser may be configured to cause switching of the switch between the first state and the second state in dependence on detection of inhalation. Thus, the plurality of droplet ejectors may only be activated as the user inhales. This ensures that the entirety of the generated aerosol will be inhaled by the user, thereby reducing excess generation of aerosol.

The nebuliser may further comprise a controller. The controller is to (e.g. configured to) control operation of the plurality of droplet ejectors. The control of the operation of the plurality of droplet ejectors may be in response to a sensor input, such as a state of a switch.

The controller may comprise one or more processors and a memory configured to store instructions which when executed by the one or more processors cause the nebuliser to carry out the functions of the controller described herein. The memory may be non-transitory, computer readable memory. The memory may have the instructions stored thereon. The present invention extends to a non-transitory computer-readable medium (e.g. memory) having the instructions stored thereon to control the nebuliser as described herein. The memory may be solid-state memory. The controller may be provided in a single device. In other example, the controller may be distributed, having a plurality of processors. A first processor may be separated from a second processor in a distributed manner. Where the controller is distributed over multiple separate devices, the nebuliser may be a nebuliser apparatus, formed from a plurality of separate devices.

In some examples, the nebuliser may further comprise an electronic display screen (e.g. an LCD display screen) for displaying information relating to the nebuliser. The electronic display screen may be a touch screen for receiving one or more control inputs for controlling operation of the nebuliser. The information relating to the nebuliser may comprise at least one of device status, sensor outputs, timers and dosing programs.

At least one nozzle outlet of the plurality of nozzle outlets may have a maximum cross-sectional extent less than 20 × 10⁻⁶ metres (20 microns). More than 50 percent of the plurality of nozzle outlets may have a maximum cross-sectional extent less than 20 × 10⁻⁶ metres (20 microns). Each of the plurality of nozzle outlets may have a maximum cross-sectional extent less than 20 × 10⁻⁶ metres (20 microns).

At least one nozzle outlet of the plurality of nozzle outlets may have a maximum cross-sectional extent less than 15 × 10⁻⁶ metres (15 microns). More than 50 percent of the plurality of nozzle outlets may have a maximum cross-sectional extent less than 15 × 10⁻⁶ metres (10 microns). Each of the plurality of nozzle outlets may have a maximum cross-sectional extent less than 15 × 10⁻⁶ metres (10 microns).

At least one nozzle outlet of the plurality of nozzle outlets may have a minimum cross-sectional extent greater than 5 × 10⁻⁶ metres (5 micron). More than 50 percent of the plurality of nozzle outlets may have a minimum cross-sectional extent greater than 5 × 10⁻⁶ metres (5 micron). Each of the plurality of nozzle outlets may have a minimum cross-sectional extent greater than 5 × 10⁻⁶ metres (5 micron).

At least one nozzle outlet of the plurality of nozzle outlets may have a minimum cross-sectional extent greater than 8 × 10⁻⁶ metres (8 microns). More than 50 percent of the plurality of nozzle outlets may have a minimum cross-sectional extent greater than 8 × 10⁻⁶ metres (8 microns). Each of the plurality of nozzle outlets may have a minimum cross-sectional extent greater than 8 × 10⁻⁶ metres (8 microns).

At least one nozzle outlet of the plurality of nozzle outlets may have a cross-sectional area less than 2 × 10⁻¹⁰ m². More than 50 percent of the plurality of nozzle outlets may have a cross-sectional area less than 2 × 10⁻¹⁰ m². Each of the plurality of nozzle outlets may have a cross-sectional area less than 2 × 10⁻¹⁰ m².

At least one nozzle outlet of the plurality of nozzle outlets may have a cross-sectional area less than 1 × 10⁻¹⁰ m². More than 50 percent of the plurality of nozzle outlets may have a cross-sectional area less than 1 × 10⁻¹⁰ m². Each of the plurality of nozzle outlets may have a cross-sectional area less than 1 × 10⁻¹⁰ m².

At least one nozzle outlet of the plurality of nozzle outlets may have a cross-sectional area greater than 1 × 10⁻¹¹ m² (0.5 µm²). More than 50 percent of the plurality of nozzle outlets may have a cross-sectional area greater than 1 × 10⁻¹¹ m². Each of the plurality of nozzle outlets may have a cross-sectional area greater than 1 × 10⁻¹¹ m².

At least one nozzle outlet of the plurality of nozzle outlets may have a cross-sectional area greater than 5 × 10⁻¹¹ m² (0.5 µm²). More than 50 percent of the plurality of nozzle outlets may have a cross-sectional area greater than 5 × 10⁻¹¹ m². Each of the plurality of nozzle outlets may have a cross-sectional area greater than 5 × 10⁻¹¹ m².

Typically, each of the nozzle outlets will have a substantially similar cross-sectional shape. The cross-sectional shape of the nozzle outlets may be rounded, such as circular.

It will be understood that the nozzle outlet is where the one or more droplets are ejected from the nozzle portion of the droplet ejector. Thus, references to the cross-sectional extent or cross-section area of the nozzle outlet refer to the region of the nozzle portion which is last in contact with the liquid to be ejected as one or more droplets before the droplet is released from the droplet ejector.

**At least** 50% of the droplets of the aerosol may have a volume of at least 5 × 10⁻¹⁷ m³. At least 50% of the droplets of the aerosol may have a volume of at least 2 × 10⁻¹⁶ m³, optionally at least 3 × 10⁻¹⁶ m³.

At least 50% of the droplets of the aerosol may have a volume no greater than 2 × 10⁻¹⁵ m³, for example less than 1 × 10⁻¹⁵ m³, optionally no greater than 7 × 10⁻¹⁶ m³.

At least 80% of the droplets of the aerosol may have a volume of at least 5 × 10⁻¹⁷ m³. At least 80% of the droplets of the aerosol may have a volume of at least 2 × 10⁻¹⁶ m³, optionally at least 3 × 10⁻¹⁶ m³.

At least 80% of the droplets of the aerosol may have a volume no greater than 2 × 10⁻¹⁵ m³, for example less than 1 × 10⁻¹⁵ m³, optionally no greater than 7 × 10⁻¹⁶ m³.

At least 90% of the droplets of the aerosol may have a volume of at least 5 × 10⁻¹⁷ m³. At least 90% of the droplets of the aerosol may have a volume of at least 2 × 10⁻¹⁶ m³, optionally at least 3 × 10⁻¹⁶ m³.

At least 90% of the droplets of the aerosol may have a volume no greater than 2 × 10⁻¹⁵ m³, for example less than 1 × 10⁻¹⁵ m³, optionally no greater than 7 × 10⁻¹⁶ m³.

At least 95% of the droplets of the aerosol may have a volume of at least 5 × 10⁻¹⁷ m³. At least 95% of the droplets of the aerosol may have a volume of at least 2 × 10⁻¹⁶ m³, optionally at least 3 × 10⁻¹⁶ m³.

At least 95% of the droplets of the aerosol may have a volume no greater than 2 × 10⁻¹⁵ m³, for example less than 1 × 10⁻¹⁵ m³, optionally no greater than 7 × 10⁻¹⁶ m³.

At least 99% of the droplets of the aerosol may have a volume of at least 5 × 10⁻¹⁷ m³. At least 99% of the droplets of the aerosol may have a volume of at least 2 × 10⁻¹⁶ m³, optionally at least 3 × 10⁻¹⁶ m³.

At least 99% of the droplets of the aerosol may have a volume no greater than 2 × 10⁻¹⁵ m³, for example less than 1 × 10⁻¹⁵ m³, optionally no greater than 7 × 10⁻¹⁶ m³.

Each of the droplets of the aerosol may have a volume of at least 5 × 10⁻¹⁷ m³. Each of the droplets of the aerosol may have a volume of at least 2 × 10⁻¹⁶ m³, optionally at least 3 × 10⁻¹⁶ m³.

Each of the droplets of the aerosol may have a volume no greater than 2 × 10⁻¹⁵ m³, for example less than 1 × 10⁻¹⁵ m³, optionally no greater than 7 × 10⁻¹⁶ m³.

The plurality of droplet ejectors may be formed on a common substrate. In other words, both the nozzle portions and the piezoelectric actuators of all of the plurality of droplet ejectors may be formed on the common substrate.

Typically, the substrate has a first surface and an opposite second surface. The substrate comprises a CMOS control circuit, a plurality of layers on the first surface of the substrate, the piezoelectric actuator being formed by one or more said layers and the nozzle portion defining a hole (e.g. the nozzle outlet) through the one or more said layers, such that the piezoelectric actuator displaces the one or more said layers and the nozzle portion in use to thereby eject droplets. Thus, the droplet ejectors are typically configured to eject droplets in an inertial mode. It may be that the hole extends through the piezoelectric actuator.

The piezoelectric actuator typically operates to cause displacement of a resiliently deformable membrane defining at least a portion of the nozzle outlet in such a way as to cause ejection of the droplets from said nozzle portion on operation of said actuator. In this way, it can be considered that the resiliently deformable membrane is part of the nozzle portion.

In this way, typically the piezoelectric actuator is coupled to the respective nozzle portion. Thus, deformation of the piezoelectric actuator during use leads to movement of a region of the nozzle portion, in turn causing ejection of the droplets from the nozzle outlet. Typically, the resiliently deformable membrane comprises the piezoelectric actuator and defines at least a region of the nozzle portion. Thus, the resiliently deformable membrane defines at least part (e.g. a wall) of a chamber in fluid communication with the nozzle outlet. Typically, the piezoelectric actuator is disposed adjacent to, for example around, the nozzle outlet. Typically, actuation of the piezoelectric actuator deflects the resiliently deformable membrane, which defines the nozzle outlet. Thus, the resiliently deformable membrane and nozzle portion move in use to eject droplets from within the chamber out through the nozzle outlet. Typically, the nebuliser comprises a nozzle-defining layer formed on the substrate, the nozzle-defining layer comprising the piezoelectric actuator and defining the nozzle outlet. The nozzle-defining layer typically comprises at least one piezoelectric layer and one or more electrodes in electrical contact with the at least one piezoelectric layer.

In other possible arrangements, the piezoelectric actuator is in distal association with the nozzle outlet, and/or the nozzle outlet and piezoelectric actuator are on different walls of the chamber (e.g. the nozzle outlet is on one wall of the fluid chamber, and the actuator is on the opposing wall of the same chamber such that the piezoelectric actuator is far away from the nozzle outlet). Where the piezoelectric actuator is not coupled to the nozzle outlet/is away from the nozzle outlet requires a large actuation force to compress almost the entire liquid stored in the chamber in order to eject the droplets through the nozzle outlet. This operation thus depends upon compressibility mode. By contrast, the use of the inertial mode avoids disturbing the majority of the liquid in the chamber, and only requires a small actuation force to displace the liquid in the nozzle outlet. The liquid is then ejected as a droplet mainly by inertial force (i.e. inertial ejection) from the nozzle outlet. Herein, ejection by inertial force can also be referred to as inertial ejection or ejection by inertial mode. Where the piezoelectric actuator is coupled to the respective nozzle portion, ejection of the liquid (from the respective nozzle) is permitted by inertial mode instead of by compressibility mode.

It will be understood that ejection by inertial mode (i.e. inertial ejection) has a number of closely associated benefits. It permits the use of low-temperature processable piezoelectric materials having lower piezoelectric constants (i.e. piezoelectric materials which are processable below 450 degrees Celsius or below 300 degrees Celsius) since only a small actuation force is initially required. The small force exerted by a piezoelectric actuator comprising low-temperature processable piezoelectric materials gives relatively low fluid pressure such that an acoustic cross talk problem (i.e. neighbouring actuators and fluid chambers interact with one another through pressure waves in the fluid) is partially or even completed mitigated. Lower levels of acoustic cross talk in turn permit close integration of neighbouring ejectors for a compact configuration of the nebuliser.

Specifically in the context of medications, it is often the case that the substances to be nebulised are chemically delicate, and cannot be subjected to excessive heat, strain or pressure, so as to retain their efficacy. Ejection by inertial mode is particularly suited to such constraints due to the relatively low inertial forces required to generate the aerosol. As a result, the liquid comprising the substance is not significantly heated or otherwise altered during the ejection process. Accordingly, inertial mode ejection is well suited to nebulising a wide range of substances, for example medications, including aqueous solutions, solid suspensions, proteins and peptides.

It may be that all of the plurality of droplet ejectors for the nebuliser are provided on a single droplet ejector chip, for example on a single substrate.

The nozzle portion and the piezoelectric actuator for each droplet ejector may be formed together by fabrication.

The nebuliser may further comprise driving circuitry to control operation of the piezoelectric actuator responsive to control signals received thereat. Thus, the piezoelectric actuators can be individually activated by the driving circuitry. The driving circuitry may be fabricated at least partially with the piezoelectric actuators. Thus, costs of manufacture for the nebuliser can be advantageously constrained because the multiple components required to both provide and allow control of the plurality of droplet ejectors can be formed together, rather than each droplet ejector needing to be formed separately, or the driving circuitry needing to be separately attached to a pre-formed plurality of droplet ejectors.

The plurality of droplet ejectors may be at least four droplet ejectors. The plurality of droplet ejectors may be at least 25 droplet ejectors. The plurality of droplet ejectors may be at least 100 droplet ejectors. The plurality of droplet ejectors may be fewer than 10,000 droplet ejectors. The plurality of droplet ejectors may be fewer than 1,000 droplet ejectors. Thus, aerosols having relatively high densities of droplets can be generated.

The plurality of droplet ejectors may be arranged such that the nozzle outlets are provided in a grid arrangement. Thus, the generated droplets have a distribution which is particularly simple to predict, making it simpler to generate aerosols having a desired set of properties, such as droplet density.

It may be that the plurality of droplet ejectors are arranged such that the nozzle outlets are provided in a regular arrangement. The plurality of droplet ejectors may define a substantially rectilinear arrangement, such as a rectangular or square arrangement. In some examples, the plurality of droplet ejectors may define a substantially quadrilateral arrangement, such as arranged substantially in a parallelogram.

The nebuliser further comprises a signal generator configured to generate a driving signal having a repeating waveform, and driving circuitry configured to relay the driving signal to the piezoelectric actuators via a plurality of switches and to control the plurality of switches to selectively apply the driving signal to individual piezoelectric actuators to thereby eject droplets of the liquid. It may be that the driving signal can be determined so as to cause the piezoelectric actuators to eject droplets of different volumes and/or velocities as indicated by control signals.

Suitably, the driving circuitry configured to relay the driving signal is comprised in the nebuliser. In other words, the driving circuitry can be part of the nebuliser. Furthermore, the complexity of wiring connections in the nebuliser can be reduced, because separate control wiring to each piezoelectric actuator need only be provided from the driving circuitry, rather than being provided into the nebuliser; the control instructions for any piezoelectric actuators in a nebuliser can be provided to the nebuliser via a single wiring connection to the driving circuitry on the nebuliser (should these need to be externally received).

The driving circuitry may comprise a CMOS circuit (i.e. a complementary metal oxide semiconductor) and a plurality of circuit elements each associated with a droplet ejector.

The driving circuitry may be integrally formed with the nozzle portions. In other words, formation of the driving circuitry, the nozzle portions and (optionally) the piezoelectric actuators can be provided at the same time, without requiring assembly of multiple component parts assembled separately. By providing the driving circuitry using an integrated circuit, the driving circuitry can be provided adjacent the nozzle portions, thereby ensuring the nebuliser is compact.

It may be that the driving circuitry comprises (a) a digital register. It may be that the driving circuitry comprises (b) a voltage trimming calculation circuit and/or register. It may be that the driving circuitry comprises (c) a temperature measurement circuit. It may be that the driving circuitry comprises (d) a fluid chamber fill detection circuit.

The digital register may be a shift register, or a latch register, for example. In operation, it may be that data is stored in or read from a register within the driving circuitry. In operation, it may be that temperature is measured using a temperature sensitive component of the temperature measurement circuit. In operation, it may be that the fill level of a fluid chamber is measured.

It may be that the driving circuitry is configured to modify the voltage pulses applied to one or more electrodes of one or more piezoelectric actuators responsive to data stored by the driving circuitry or measurements from one or more sensors, which are typically within the nebuliser. In operation, it may be that the driving circuitry measures the voltage pulses applied to one or more electrodes of one or more piezoelectric actuators responsive to data stored by the driving circuitry or measurements from one or more sensors, which are typically within the nebuliser.

Modifying the voltage pulses may comprise shifting them in time. Modifying the voltage pulses may comprise compressing or expanding them. Modifying the voltage pulses may comprise modifying their magnitude. Modifying the voltage pulses may comprise swapping between a plurality of (typically repeating) sequences of received actuator drive pulses with different profiles. The driving circuitry is typically configured to modify the voltage pulses applied to one or more electrodes of one or more individual piezoelectric actuators responsive to data relating to that individual piezoelectric actuator stored by the driving circuitry or measurements from one or more sensors.

The driving circuitry comprises a plurality of circuit elements each associated with a droplet ejector. The circuit element can be an ejection transistor. The ejection transistor is typically in direct electrical communication (without intervening switched semiconductor junction) with an electrode of the piezoelectric actuator. In operation, it may be that the ejection transistor is controlled to cause a potential output from the ejection transistor to be applied directly to an electrode of the piezoelectric actuator.

The driving circuitry may be configured to receive input control signals from outside the plurality of droplet ejectors, such as from outside the nebuliser and to output actuator control signals to each of the plurality of piezoelectric actuators to control ejection of the droplets from the nozzle outlets of the plurality of droplet ejectors.

It may be that the nebuliser further comprises an electrical input for receiving actuator drive pulses. In operation, the nebuliser may receive actuator drive pulses.

The nebuliser may comprise a controller for controlling the nebuliser. The controller may comprise one or more microcontrollers or microprocessors, which may be integrated or distributed, in communication with or comprising a memory storing program code.

It may be that the controller comprises a signal generator configured to generate (typically a sequence of) actuator drive pulses. Each nebuliser typically comprises an electrical input connected to the controller through which the actuator drive pulses are received. In operation, a nebuliser assembly may generate actuator drive pulses (e.g. in a controller) and conduct them to the nebuliser through an electrical connection.

The actuator drive pulses are typically analogue signals. The actuator drive pulses typically comprise periodic repeating voltage waveforms.

It may be that the driving circuitry is configured to switchedly connect or disconnect at least one electrode of the or each of a plurality of piezoelectric actuators to the received actuator drive pulses to thereby selectively actuate the piezoelectric actuators. In operation, it may be that the nebuliser switchedly connects or disconnects at least one electrode of the or each of a plurality of piezoelectric actuators to the received actuator drive pulses to thereby selectively actuate the piezoelectric actuators.

It may be that the controller comprises one or more pulse generators which generate a plurality of sequences of actuator drive pulses, and electrical inputs of the plurality of droplet ejectors receive the plurality of sequences of actuator drive pulses (generated by the one or more pulse generators) through a plurality of electrical connections to the controller, and the driving circuitry is configured to switchedly connect or disconnect at least one electrode of the or each of a plurality of piezoelectric actuators to received actuator drive pulses selected from a plurality of different received sequences of actuator pulses. In operation, it may be that the nebuliser generates a plurality of different sequences of actuator drive pulses (e.g. in a controller) and conducts them to the plurality of droplet ejectors through separate electrical connections, and switchedly connects or disconnects at least one electrode of the or each of a plurality of piezoelectric actuators to one or more received actuator drive pulses received from a variable (and selectable) one of the plurality of different sequences of actuator drive pulses.

The selection as to which received sequence of actuator pulses at least one electrode of the piezoelectric actuator is connected to may be responsive to stored data specific to the respective piezoelectric actuator and/or responsive to measurements of operation of the respective piezoelectric actuator. Accordingly, the driving circuitry can typically select whether or not each piezoelectric actuator ejects a droplet at each of a sequence of periodic droplet ejection decision points. By a decision point we refer to a time prior to the start of an actuator drive pulses where it is determined whether or not to communicate that actuator drive pulse to at least one electrode of a specific piezoelectric actuator.

Typically, the actuator drive pulses repeat periodically. It may be that the actuator drive pulses are amplified by the controller. It may be that the actuator drive pulses are not amplified by the nebuliser. It may be that the nebuliser does not generate actuator drive pulses.

Typically, pulses from the pulse generator are conducted to a plurality of control circuits. Thus a single pulse generator circuit may drive multiple piezoelectric transducers on the same substrate.

The digital actuation control signals are typically received from a controller. The digital actuation control signals are typically received through one or more flexible connectors. The digital actuation control signals may be received in serial form and converted to parallel control signals using a shift register within the driving circuitry.

It may be that the controller comprises a pulse generator configured to generate actuator drive pulses which are conducted to the plurality of droplet ejectors and digital control signals which are conducted to the plurality of droplet ejectors and the digital control signals are processed in the driving circuitry of the nebuliser to determine which actuator drive pulses are conducted to at least one electrode of the piezoelectric actuators of the plurality of droplet ejectors to cause droplet ejection.

In operation, it may be that the nebuliser generates actuator drive pulses (e.g. at a controller) and digital control signals, and conducts both the actuator drive pulses and the digital control signals to the driving circuitry of the nebuliser and the driving circuitry processes the digital control signals and, responsive thereto, conducts selected actuator drive pulses to at least one electrode of the piezoelectric actuators of the plurality of droplet ejectors to cause droplet ejection.

Thus, typically analogue actuator drive pulse and digital control signals are input by the driving circuitry. Typically the digital control signals are used to selectively switch the analogue actuator drive pulses to thereby selectively transmit them to the piezoelectric actuators.

In some embodiments, the driving circuitry is configured to switchedly connect one or more of ground and a single fixed non-zero voltage line, or multiple fixed voltage lines of different voltages (one or more of which may be ground) to one or both electrodes of a piezoelectric actuator to cause droplet ejection of liquid. For example, the driving circuitry may switch an electrode between a connection to ground and a connection to a fixed voltage or multiple fixed voltage lines of different voltages and back to ground again in order to cause a droplet ejection.

Switching an electrode between a connection to ground and a connection to a fixed voltage or between fixed voltage lines may comprise operating a latch.

It may be that the driving circuitry is configured to individually and selectively actuate at least double the number of piezoelectric actuator elements than signal conductors through which the driving circuitry receives actuation control signals.

It may be that the said driving circuitry is configured to individually and selectively actuate at least 128 (or at least 256) piezoelectric actuator elements and the driving circuitry receives actuation control signals through at most 64 (or at most 32) signal conductors.

The driving circuitry may comprise a serial to parallel conversion circuit configured to convert a digital signal received in serial form through one or more signal conductors into a selection of piezoelectric actuators to be actuated to carry out a droplet ejection simultaneously (i.e. in parallel). The serial to parallel conversion circuit typically comprises one or more shift registers.

The nebuliser may comprise the one or more liquids.

The present disclosure further extends to a liquid adapted for use with the nebuliser described herein. Specifically, the liquid may be a medication. The disclosure extends to a refill capsule for use with the nebuliser described herein. The refill capsule may comprise the liquid, such as (or comprising) the medication.

The present disclosure also extends to a method of controlling a nebuliser as described herein. The nebuliser is substantially as described hereinbefore. The method comprises: receiving a control signal indicative of a demand to start nebulisation; and causing ejection of a plurality of droplets of liquid from the nebuliser. The ejection of the plurality of droplets is caused by activation of the plurality of droplet ejectors, for example, by applying the driving waveform described herein (or another driving waveform) to the piezoelectric actuators of a plurality of the plurality of droplet ejectors. In another aspect, the present disclosure can also further be considered to extend to a method of generating an aerosol from a liquid, using a nebuliser as described herein. The method may be a method of generating an medication-containing aerosol from a liquid comprising the medication. The method may be a method of delivering a substance to the olfactory region (e.g. of an animal, such as a human).

Where the present disclosure describes a certain proportion of the droplets having a particular property, this proportion may be by mass, or as a proportion of the total number of the droplets. The present disclosure envisages each option to be disclosed separately for every instance, and that all combinations are thereby disclosed.

### Description of the Drawings

An example embodiment of the present invention will now be illustrated with reference to the following Figures in which:
Figures 1 and 2 show a perspective view and a cross-sectional view respectively of a nebuliser in accordance with an embodiment of the present invention;
Figure 3 shows a perspective view of a nebuliser chip in accordance with an embodiment of the present invention;
Figure 4 shows a top-down view of a portion of the nebuliser chip of Figure 3, focusing on a subset of the plurality of droplet ejectors of the nebuliser chip of Figure 3;
Figure 5 shows a perspective cross-sectional view of one of the droplet ejectors shown in Figure 4;
Figure 6 is a schematic diagram showing an arrangement of a piezoelectric actuator, nozzle portion and driving circuitry as disclosed herein;
Figures 7(a) and 7(b) show actuator states for a piezoelectric actuator as used in an example of the present invention;
Figure 8 shows a possible drive waveform for driving a piezoelectric actuator in accordance with an example of the present invention;
Figure 9 shows a simplified manufacturing process flow for forming a droplet ejector in accordance with an example of the present invention;
Figure 10 shows a system diagram for a nebuliser according to an example of the present invention; and
Figure 11 shows a flowchart illustrating a method of operating a nebuliser according to an example of the present invention.

### Detailed Description of an Example Embodiment

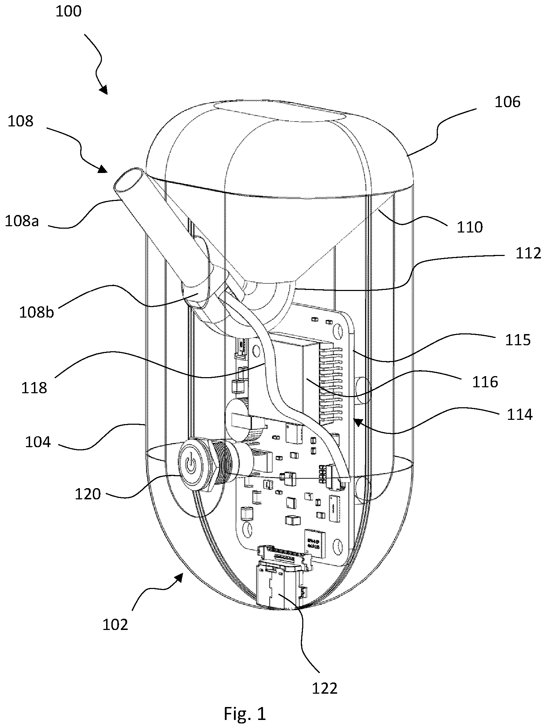

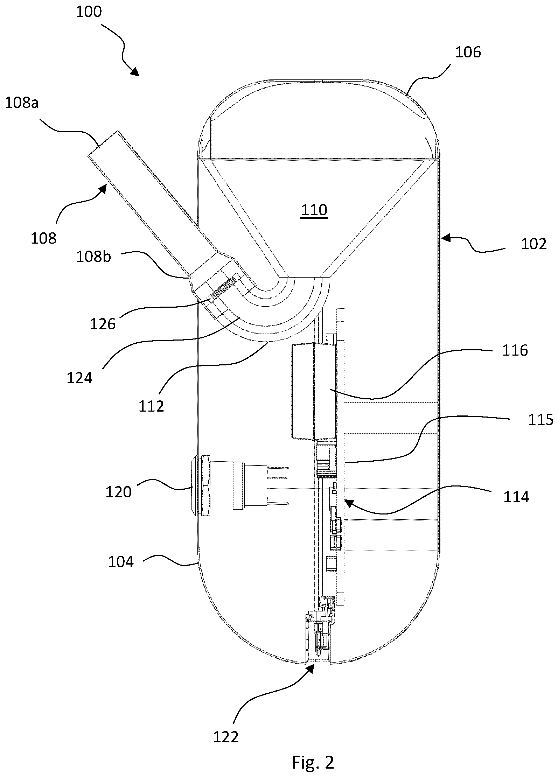

Figure 1 shows a perspective view of a nebuliser in accordance with an embodiment of the present invention. Figure 2 shows a cross-sectional view of the nebuliser shown in Figure 1. The nebuliser 100 comprises a housing 102 enclosing internal components of the nebuliser 100. The housing comprises a front portion in the form of a front side wall 104, and an upper portion 106. The upper portion 106 of the housing 102 is movable (specifically, removable) relative to the front portion 104 of the housing so as to expose an opening of a cavity 110 (best illustrated in Figure 2). The cavity 110 can receive and store a liquid therein for supplying an input of the nebuliser 100. The nebuliser 100 is further provided with an outlet portion in the form of a nose insert 108. The nose insert 108 comprises an external portion 108a and an internal portion 108b. The external portion 108a extends outwardly through the front portion 104 of the housing 102 from the internal portion 108b. The nose insert extends upwards at an angle to allow for comfortable insertion into the nose. The internal portion 108b sits inwardly within the housing 102 of the nebuliser 100 from the external portion 108a. In this way, it will be understood that a volume defined within the external portion 108a and the internal portion 108b provide an outlet channel (which can also be referred to as a nose insert channel).

The nose insert portion 108 is in fluid communication with the cavity 110 via a droplet ejector portion 112. As shown in Figure 2, the droplet ejector portion 112 comprises a liquid channel 124 directing liquid from the cavity 110 towards a nebuliser chip 126 comprising a plurality of droplet ejectors (also part of the droplet ejector portion 112, though not shown in Figures 1 and 2). On operation of the plurality of droplet ejectors, a plurality of droplets is generated from the liquid in the cavity 110 and the liquid channel 124 and are ejected into the internal portion 108b of the mouthpiece 108. The plurality of droplet ejectors are described further with reference to further Figures hereinafter.

The nebuliser 100 further comprises control circuitry 114 configured to provide control signals to the plurality of droplet ejectors to control nebulisation thereby. The control circuitry typically comprises a circuit board 115 on which electronic components are mounted, including a processor 116 in the form of a microprocessor 116, such as an integrated circuit chip 116. The control signals for the plurality of droplet ejectors are communicated from the control circuitry 114 via a wired connection 118. As will be described further hereinafter, the nebuliser chip 126 may itself include further control circuitry to convert the control signals received from the control circuitry 114 via the wired connection 118, into individual control signals to directly control each respective droplet ejector. Specifically, it may be that each droplet ejector comprises a dedicated control circuit such that a plurality of control circuits are provided on the nebuliser chip 126.

The nebuliser 100 further comprises a switch 120 mounted on the front portion 104 of the housing 102, and an external electrical connector 122 also mounted on the housing 102. Although not shown, the switch 120 and the electrical connector 122 are each electrically connected to the control circuitry 114.

It will be understood that, in the present example, one or more air inlets (not shown) will be provided at the internal portion 108b of the nose insert 108 and in fluid communication with the external environment. In this way, when the nose insert 108 is inserted into the nose, and the user inhales through the nose, air can flow into the nose insert 108 via the one or more air inlets, as will be described further hereinafter.

The operation of the nebuliser 100 to cause a substance to be delivered to the olfactory region of the nose of a user, in the form of droplets of medication, will now be described. For a pre-filled nebuliser 100, having liquid medication provided in the cavity 110, the nebuliser 100 is held in an upright position by the user with the front portion 104 of the housing 102 facing towards the user. In this way, the liquid medication is able to enter the droplet ejector portion 112 via the liquid channel 124 such that the liquid medication is in contact with a side of the nebuliser chip 126 exposed to the liquid channel 124. The user inserts the external portion 108a of the nose insert into their nose, and activates the nebuliser 100 via electrical switch 120 to cause operation of the control circuitry 114 in an operating configuration. In the operating configuration, the control circuitry 114 communicates control signals to the plurality of droplet ejectors to cause them to eject a plurality of droplets of the liquid medication into the internal portion 108b of the nose insert 108. At the same time, the user inhales which draws an airflow through the nose insert 108 (the air being supplied into the nose insert 108 via the one or more air inlets). Nebulised droplets, ejected into the nose insert 108 from the plurality of droplet ejectors, are entrained within the airflow, and therefore travel from the nose insert 108 into the nose of the user via the external portion 108a of the mouthpiece 108. The size of the droplets means they remain in the olfactory region of the nose, rather than entering the lungs of the user. It will be appreciated that in some configurations, the droplet ejectors are situated within the nose insert, and thus droplets are ejected directly towards the olfactory region, without the need for the user to inhale. This configuration reduces the chance of droplets entering the lungs.

The user will not typically need to remove the nose insert 108 from their nose to exhale into the external environment, instead the user will typically exhale through their mouth. In alternative configurations where no inhalation is required, the user may continue to breathe normally through their mouth throughout use Once a sufficient dose has been administered, the user can then deactivate the nebuliser 100 using the switch 120. Alternatively, it may be that the nebuliser 100 includes a flow sensor (not shown) and that the control circuitry 114 is configured to cause the nebuliser 100 to deactivate automatically when no inhalation is detected through the nose insert for a predetermined amount of time. In another alternative configuration, a predetermined dosage of the medication may be inserted into the nebuliser, and the control circuitry 114 may be configured to automatically deactivate the nebuliser when the volume of liquid within the cavity 110 is detected as having fallen below a threshold.

It will be understood that the control signals sent from the control circuitry 114 to the nebuliser chip 126 may be determined based on a desired dosing regimen for the liquid medication stored within the cavity 110. the desired dosing regimen (or data indicative thereof) is typically communicated to the control circuitry 114 via the electrical connector 122. The nebuliser 100 shown in Figures 1 and 2 is refillable, though it will be understood that non-refillable (i.e. single use) versions may also be desirable in certain applications. To refill the medication in the nebuliser 100, the upper portion 106 of the housing 102 is partially or even fully removed to allow further medication to be added to the cavity 110.

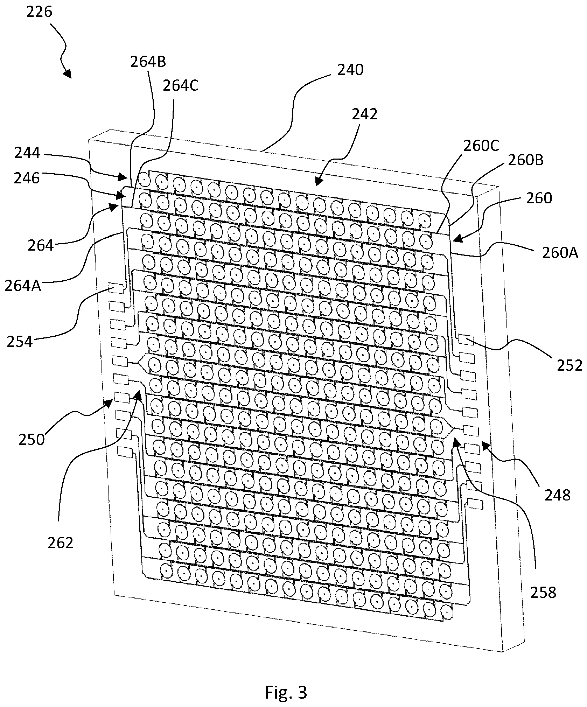

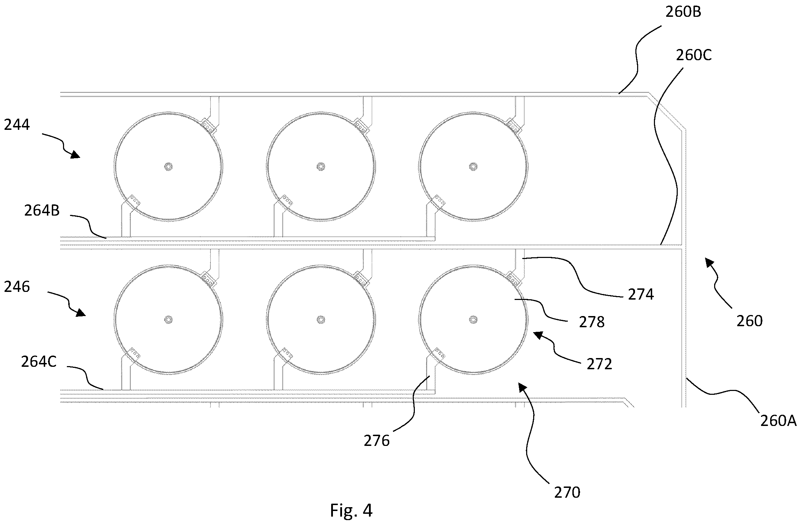

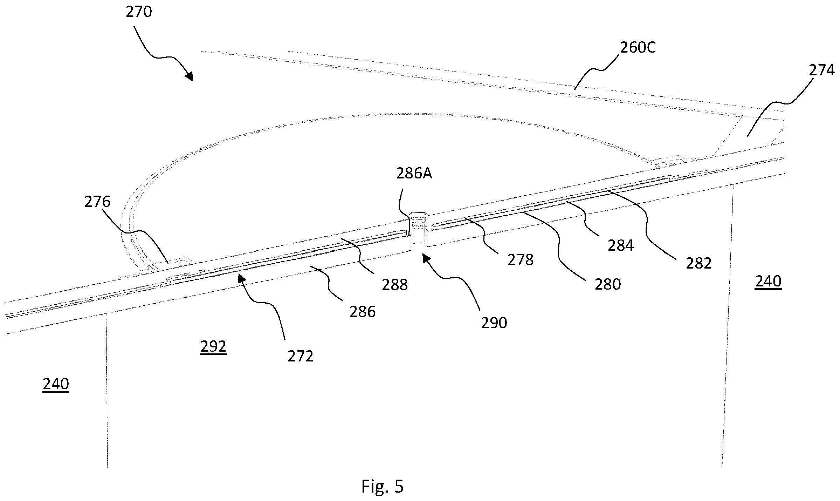

Figure 3 shows a perspective view of a nebuliser chip in accordance with an embodiment of the present invention. Figure 4 shows a top-down view of a portion of the nebuliser chip of Figure 3, focusing on a subset of the plurality of droplet ejectors of the nebuliser chip of Figure 3. Figure 5 shows a perspective cross-sectional view of one of the droplet ejectors shown in Figure 4. The nebuliser chip 226 shown in Figures 3 to 5 is an example of the nebuliser chip 126 of Figures 1 and 2.

The nebuliser chip 226 comprises a substrate 240 in the form of a silicon substrate 240 with a plurality of droplet ejectors 242 situated thereon. The plurality of droplet ejectors 242 are arranged in a plurality of rows, including a first row 244 and a second row 246. Each row is parallel to each and every other row, and is mutually aligned, such that the plurality of rows of droplet ejectors 242 are arranged in a rectangular grid.

The nebuliser chip 226 further comprises a plurality of lower portion bond pads 248 arranged in a line running perpendicular to the rows of the plurality of droplet ejectors 242 and situated adjacent to the plurality of droplet ejectors 242 on a first side thereof to communicate control signals between the plurality of droplet ejectors 242 and further control circuitry located separate to the nebuliser chip 226 (not shown in Figure 3). The nebuliser chip 226 further comprises a plurality of upper portion bond pads 250 arranged in a line running parallel to the plurality of lower portion bond pads 248 and situated adjacent to the plurality of droplet ejectors 242 on a second side thereof. The plurality of upper portion bond pads 250 are spaced from the plurality of lower portion bond pads 248. The plurality of upper portion bond pads 250 are to communicate control signals between the plurality of droplet ejectors 242 and further control circuitry located separate to the nebuliser chip 226 (not shown in Figure 3). The first and second sides are opposite to one another such that the rows of the plurality of droplet ejectors 242 extend from the first side to the second side. The plurality of lower portion bond pads 248 includes a first lower portion bond pad 252 to communicate control signals to the first two rows 244, 246 of the plurality of droplet ejectors 242 and the further control circuitry located separate to the nebuliser chip 226 (not shown in Figure 3). The plurality of upper portion bond pads 250 comprises a first upper portion bond pad 254 to communicate control signals between the first two rows 244, 246 of the plurality of droplet ejectors 242 and the further control circuitry located separate to the nebuliser chip 226 (not shown in Figure 3).

The nebuliser chip 226 further comprises a plurality of lower portion interconnects 258 extending between the plurality of lower portion bond pads 248 and the plurality of droplet ejectors 242 and providing electrical communication therebetween. In this way, control signals and/or power signals received at the plurality of lower portion bond pads 248 can be communicated to the plurality of droplet ejectors 242. Similarly, feedback signals from the plurality of droplet ejectors 242 can be communicated to the plurality of lower portion bond pads 248 via the plurality of lower portion interconnects 258. The plurality of lower portion interconnects 258 comprises a first lower portion interconnect 260 extending between the first lower portion bond pad 252 and the first and second rows 244, 246 of the plurality of droplet ejectors 242. The first lower portion interconnect 260 comprises a trunk portion 260A extending from the first lower portion bond pad 252, a first branch portion 260B extending from the aforementioned trunk portion 260A to the first row 244 of the plurality of droplet ejectors 242, and a second branch portion 260C extending from the aforementioned trunk portion 260A to the second row 246 of the plurality of droplet ejectors 242.

The nebuliser chip 226 further comprises a plurality of upper portion interconnects 262 extending between the plurality of upper portion bond pads 250 and the plurality of droplet ejectors 242 and providing electrical communication therebetween. In this way, control signals and/or power signals received at the plurality of upper portion bond pads 250 can be communicated to the plurality of droplet ejectors 242. Similarly, feedback signals from the plurality of droplet ejectors 242 can be communicated to the plurality of upper portion bond pads 250 via the plurality of upper portion interconnects 262.The plurality of upper portion interconnects 262 comprises a first upper portion interconnect 264 extending between the first upper portion bond pad 254 and the first and second rows 244, 246 of the plurality of droplet ejectors 242 providing electrical communication therebetween. The first upper portion interconnect 264 comprises a trunk portion 264A extending from the first upper portion bond pad 254, a first branch portion 264B extending from the aforementioned trunk portion 264A to the first row 244 of the plurality of droplet ejectors 242, and a second branch portion 264C extending from the aforementioned trunk portion 264A to the second row 246 of the plurality of droplet ejectors 242.

The second row 246 of the plurality of droplet ejectors 242 comprises a droplet ejector 270 comprising an actuator portion 272 (best illustrated in Figures 4 and 5) in electrical communication with the second branch portion 260C of the first lower portion interconnect 260 via a lower portion metallisation contact 274 of the droplet ejector 270 and in electrical communication with the second branch portion 264C of the first upper portion interconnect 264 via an upper portion metallisation contact 276 of the droplet ejector 270. The droplet ejector 270 further comprises an outer passivation layer 278 encasing the actuator portion 272 and providing electrical stability thereto. The lower and upper portion metallisation contacts 274, 276 are in electrical communication with the actuator portion 272 via apertures in the outer passivation layer 278 through which the lower and upper portion metallisation contacts 274, 276 extend.

The actuator portion 272 comprises a lower electrode 280 in electrical communication with the lower portion metallisation contact 274 and an upper electrode 282 in electrical communication with the upper portion metallisation contact 276 such that a potential difference can be applied across a piezoelectric layer 284 situated therebetween to cause contraction or expansion thereof, whereby to cause actuation of the actuator portion 272 in a direction transverse to a plane of the piezoelectric layer.

The droplet ejector 270 is formed upon a foundational passivation layer 286 in the form of a nozzle-defining layer 286, which defines the inner portion of a nozzle outlet 290. The external portion of the nozzle outlet 290 is defined by a protective front surface 288, provided to cover and protect the droplet ejector 270 and abutting against a surface 286A of the foundational passivation layer 286.

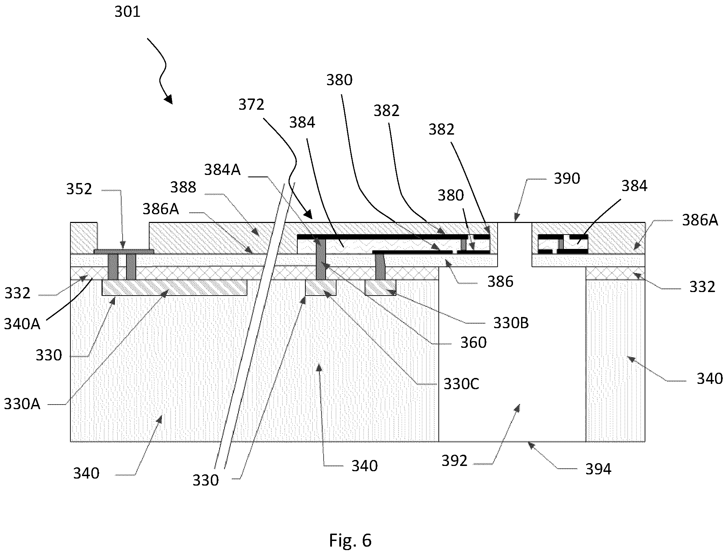

A droplet ejector cavity 292 is defined within the droplet ejector 270, in fluid communication with the nozzle outlet 290. As will be described further hereinafter with reference to Figure 6, in operation, liquid medication in the droplet ejector cavity 292 is expelled out of the nozzle outlet 290 on operation of the droplet ejector 270.

Figure 6 is a schematic diagram showing an arrangement of a piezoelectric actuator, nozzle portion and driving circuitry for a droplet ejector to be used in embodiments of the present disclosure. Although Figure 6 is substantially schematic, it will be understood that the features and operation of the droplet ejector are equally applicable to the examples described previously with reference to Figures 3 to 5 apart from those described differently hereinafter, unless inherently incompatible therewith. The droplet ejector 301 shown in Figure 6 comprises a silicon substrate 340 comprising driving circuitry 330 on the first surface 340A of the silicon substrate 340. The driving circuitry 330 is for receiving control signals and generating driving signals to control operation of the piezoelectric actuator. The driving circuitry 330 is typically an integrated circuit 330 in the form of a CMOS circuit 330. The person skilled in the art will appreciate that a CMOS circuit comprises both doped regions of the substrate and metallisation layers and interconnections formed on the first surface of the substrate. A plurality of layers shown generally as 332 are formed on the first surface 340A of the silicon substrate 340. Layer 332 is the CMOS metallisation layer and comprises metal conductive traces and a passivation insulator such as SiO₂, SiN, SiON. The droplet ejector 301 further comprises an actuator portion 372 in the form of a piezoelectric actuator 372 comprising a piezoelectric layer 384 which in this example is formed of AIN or ScAlN but may be formed of another suitable piezoelectric material which is processable at a temperature of below 450°C. The piezoelectric actuator 372 forms a diaphragm with layers of materials such as silicon, silicon oxide, silicon nitride or derivatives thereof and has a passivation layer 386 (sometimes referred to as a nozzle outlet defining layer 386, or referred to as a nozzle plate 386) which prevents applied electrical potentials from contacting fluid.

At least one metallisation layer 332 includes interconnects conducting signals from an external controller via a bond pad 352 to a first portion 330A of the driving circuitry 330 and from second and third portions 330B, 330C of the driving circuitry 330 to the piezoelectric actuator 372 via electrical interconnects 360, in particular to lower electrodes 380 and upper electrodes 382 arranged to apply an electrical potential difference across and thereby actuate the piezoelectric layer 384. An opening 384A is defined in the piezoelectric layer 384 for passage of the electrical interconnect 360 between the third portion 330C of the driving circuitry 330 and the upper electrode 382.

The piezoelectric actuator 372 and accompanying nozzle outlet defining layer 386 defines a wall of a droplet ejector cavity 392 in the form of a liquid chamber 392 which receives liquid, such as liquid comprising the substance, for example liquid medication, through a liquid chamber inlet 394 in fluid communication with at least one of the one or more inputs described hereinbefore. The liquid chamber 392 is further in fluid communication with a nozzle outlet 390 for ejecting liquid. The piezoelectric actuator 372 and the nozzle outlet defining layer 386 further define a wall of the nozzle outlet 390. The liquid chamber inlet 394 forms at least part of a liquid manifold providing a liquid communication pathway between a cavity (not shown in Figure 6) and the nozzle outlet 390 (as well as, in typical examples, further nozzle outlets, not shown in Figure 6), via the liquid chamber 392. The liquid chamber inlet 394 is defined by the silicon substrate 340, the metallisation layer 332 and the nozzle outlet defining layer 386. A protective front surface 388 provides the external surface of the droplet ejector 301, provided to cover and protect the piezoelectric actuator 372, and abutting against a surface 386A of the nozzle outlet defining layer 386. The protective front surface 388 has apertures which define the nozzle outlet 390 (as well as further nozzle outlets, not shown in Figure 6). The piezoelectric actuator 372, liquid chamber 392 and nozzle outlet 390 together form the droplet ejector 301.

Typically, the CMOS control circuit comprises patterned regions of doped silicon and metallisation layers. The number of metallisation layers depends on the complexity of the CMOS control circuit, but three layers should suffice for many applications.

Although only one nozzle outlet 390 and piezoelectric actuator 372 is shown in Figure 6 for clarity, it will be understood that a plurality of nozzle outlets 390 and corresponding piezoelectric actuators 372 are typically provided together as a plurality of droplet ejectors, but on the same substrate 340. Each piezoelectric actuator 372 is configured to control ejection of liquid from the respective nozzle outlet 390.

The droplet ejector 301 of Figure 6 ejects liquid through the nozzle outlet 390. The piezoelectric actuator 372 is in the nozzle outlet defining layer 386 which moves with the nozzle outlet 390. Thus, the surface of the liquid chamber 392 that includes the nozzle outlet 390 is the surface that moves during actuation. This contrasts with devices in which the surface that includes the nozzle outlet does not move and another surface, e.g. the opposite surface, is actuated. As a result, only a small actuation force is required to displace the liquid in the nozzle outlet portion. The liquid is then ejected mainly by inertial force (i.e. inertial ejection) from the nozzle outlet. Herein, ejection by inertial force can also be referred to as inertial ejection or ejection by inertial mode. The ejection of the liquid is dictated predominantly by the density and viscosity of the liquid - not by compressibility as would be the case if another surface was actuated and the liquid in the chamber was displaced as a whole in order to cause the ejection.

In use, the droplet ejector 301 is mounted on a support including a liquid manifold that supplies liquid to the liquid chamber inlet 394. Fluid pressure is typically slightly negative at the liquid chamber inlet 394 and the liquid chamber 392 typically "primes" or fills with liquid by surface tension driven capillary action. The nozzle outlet 390 primes up to the outer surface of the protective front surface 388 due to capillary action once the liquid chamber 392 is primed. The liquid does not move onto the outer surface of the protective front surface 388 past the nozzle outlet 390 due to the combination of negative fluid pressure and the geometry of the nozzle outlet 390.

The second and third portions 330B, 330C of the driving circuitry 330 control the application of a voltage pulse to the lower electrode 380 and upper electrode 382, respectively, according to a timing signal from the first portion 330A of the driving circuitry 330. The application of electrode voltage across the piezoelectric layer 384 creates an electric field. The application of this electric field causes a deformation of the piezoelectric layer 384. The deformation can either be tensile or compressive strain depending on the orientation of the electric field with respect to the direction of polarisation in the piezoelectric material. The induced strain caused by the expansion or contraction of the piezoelectric layer 384 induces a strain gradient through the thickness of the nozzle plate 386, piezoelectric actuator 372 and the protective front surface 388 causing a movement or displacement in a direction parallel to an axis defined by the nozzle outlet 390.

The piezoelectric properties of the piezoelectric material can be characterized in part by the transverse piezoelectric constant *d*₃₁*. d*₃₁ is the particular component of the piezoelectric coefficient tensor which relates the electric field applied across the piezoelectric material in a first direction to the strain induced in the piezoelectric material along a second direction perpendicular to said first direction. The piezoelectric actuator 372 shown is configured such that the applied electric field induces a strain in the material in a direction perpendicular to the direction in which the field is applied and is therefore characterized by the *d*₃₁ constant.

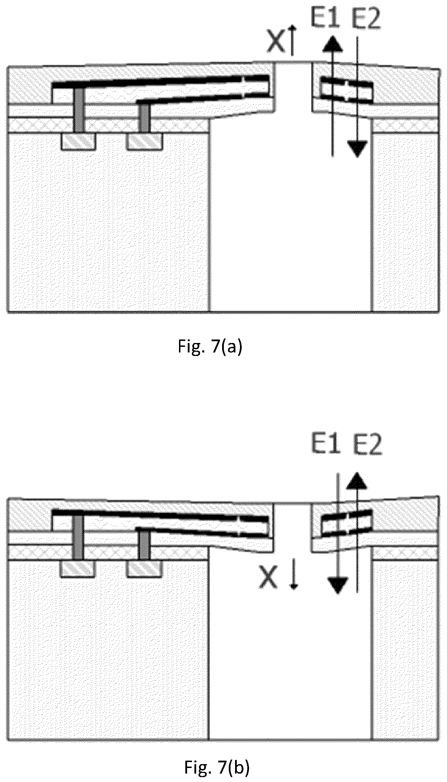

The application of a DC or constant electric field can cause a net positive or negative displacement of the nozzle plate 386. A positive displacement of the nozzle plate is shown in Figure 7(a).

The application of a pulsed electric field can cause an oscillation of the nozzle plate 386. This oscillation of the nozzle plate 386 induces a pressure in the liquid chamber 392 under the nozzle plate 386 which causes droplet ejection out of the nozzle outlet 390. The frequency and amplitude of the oscillation of the nozzle plate 386 is primarily a function of the mass and stiffness characteristics of the nozzle plate 386, the piezoelectric actuator 372, and the protective front surface 388, the properties of the liquid (for example, the density, viscosity (either Newtonian or non-Newtonian), and surface tension), the geometries of the nozzle outlet 390 and the liquid chamber 392, and the configuration of the drive pulses to the piezoelectric actuators.

Figures 7(a) and 7(b) show movement of a piezoelectric actuator. Voltage pulses are applied across lower electrode 380 and upper electrode 382 to cause the movements shown. The electric field direction is labelled as E and the deflection is labelled as X.

The application of a steady state or DC electric field across the electrodes causes a contraction in the piezoelectric layer 384 and a steady state deflection of the nozzle plate 386 away from the liquid chamber inlet 394 as shown in Figure 7(a). The fluid pressure under the nozzle plate 386 is the same as the supply pressure from the liquid chamber inlet 394. Strain energy is stored in the nozzle plate 386, the piezoelectric actuator 372 and the protective front surface 388.

Subsequently, the electric field is removed, and a reverse electric field pulse is applied. This causes both a release of the stored strain energy and the application of additional expansion of the piezoelectric material of piezoelectric layer 384. The piezoelectric actuator 372 moves towards the liquid chamber inlet 394 as shown in Figure 7(b). This causes a positive pressure in the liquid chamber inlet 394 and nozzle region which causes droplet ejection out of the nozzle outlet 390. The reverse electric field pulse may come immediately after the removal of the DC pulse or at a slightly delayed duration.

The final removal of the electric field across the piezoelectric layer 384 causes the nozzle plate 386 to return to a position with no induced strain.

The control of two electrodes for any nozzle-actuator-nozzle plate in the device facilitates directional switching of the applied electric fields in relation to the inherent polarisation of the piezoelectric material. This allows the device to incorporate stored strain energy into the nozzle plate 386 and piezoelectric actuator 372 structure. The release and integration of this stored strain energy augments volumetric displacements during a nozzle plate droplet ejection oscillation. The increased volumetric displacement is achieved without having to increase applied voltages and electric fields.

It is also possible to replace the DC electric field configuration described hereinbefore with a pulse field configuration. This has the advantage of minimizing any applied strain effects over longer durations. An additional advantage of the dual pulsed approach is enabled by the timing of the field pulse switching application. The application of the first pulse will induce an oscillation with an initial nozzle plate movement away from the liquid inlet as shown in Figure 7(a). This oscillation will introduce a negative fluid pressure under the nozzle plate which introduces a net liquid flow towards the nozzle which can additionally augment the liquid ejection flows through the nozzle.

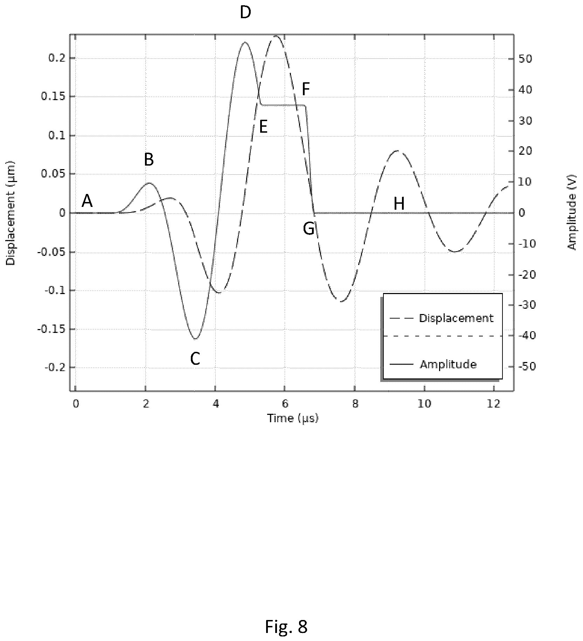

Figure 8 shows a possible drive waveform for use in driving the piezoelectric actuators described herein. The x-axis is time (in microseconds, µs), the right y-axis is the amplitude of the signal (in volts, V), and the left y-axis is the resulting displacement of the piezoelectric actuator (in micrometres, µm). The signal has an initial voltage of 0 V at point A and is initially raised to a positive potential difference peaking at point B (to cause deformation of the piezoelectric actuator in one direction), and then lowered to a negative potential difference having a trough at point C (to cause greater deformation of the piezoelectric actuator in the opposite direction). The signal is further returned to a positive potential difference having a peak at point D, before being lowered and held at a magnitude of around 35 V for approximately 1.2 µs between point E and point F. Subsequently, the signal is lowered back to 0 V at point G, and held there, as shown in point H. The use of a driving waveform of this shape causes displacement of the actuator as depicted by the broken line of Figure 8, with droplet ejection occurring between point E and point G. In this way, it can be seen that the displacement of the piezoelectric actuator follows the initial stages of the voltage amplitude of the input signal between points A to E with a slight delay, growing in amplitude as the input signal grows. The displacement of the piezoelectric actuator damps once the input signal drops to 0 at point G. Of course, it will be understood that other driving waveforms can be used, and may be different for different liquids and geometries of the droplet ejector.

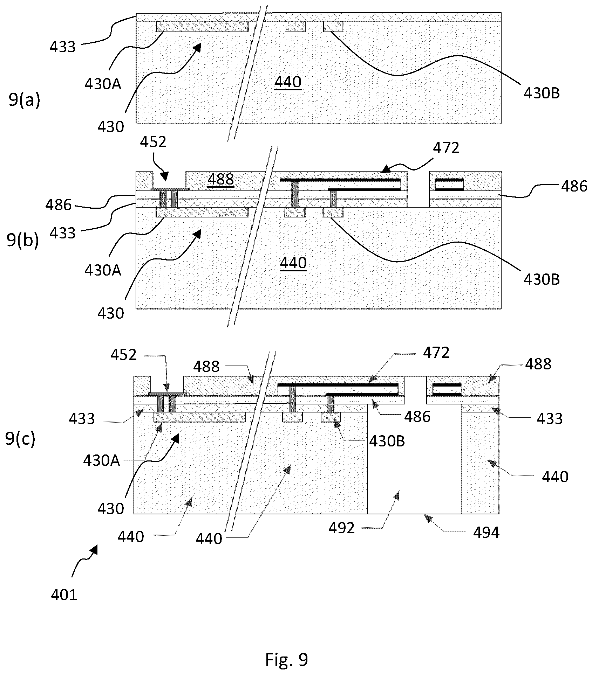

Figure 9 illustrates stages in a simplified manufacturing process flow for forming a droplet ejector in accordance with examples described herein. The droplet ejector 401 is substantially similar to the droplet ejector 301 shown in Figure 6, apart from the hereinafter described differences. Like features are illustrated with like reference numbers, with the first digit changing from 3 to 4, to indicated that the feature is relevant to Figure 9 instead of Figure 6 (e.g., the nozzle plate 386 of Figure 6 is the nozzle plate 486 of Figure 9). Specifically, the droplet ejector 401 includes a silicon substrate 440, first and second portions 430A, 430B of driving circuitry 430, a bond pad 452, a nozzle plate 486, a piezoelectric actuator 472, a protective front surface 488, a liquid chamber 492 and a liquid chamber inlet 494.

A first manufacturing step, as shown in Figure 9(a), is to create the driving circuitry 430 and an interconnect layer 433, for example CMOS driving circuitry 430 and interconnects 433, on a surface of a silicon substrate 440. The CMOS driving circuitry 430 is formed by standard processes - for example ion implantation on p-type or n-type substrates followed by the creation of a wiring interconnect layer by standard CMOS fabrication processes (e.g. ion implantation, chemical vapour deposition (CVD), physical vapour deposition (PVD), etching, chemical-mechanical planarization (CMP) and/or electroplating).

Subsequent manufacturing steps are implemented to define features and structures of the droplet ejector device. Subsequent steps are chosen not to damage structures formed in previous steps. A key manufacturing parameter is the peak processing temperature. Problems associated with processing CMOS at high temperatures include the degradation of dopant mobility and interconnect wiring schemes. CMOS electronics are known to survive temperatures of 450°C. However, a much lower temperature (i.e. below 300°C) is desirable for high yield.

The nozzle plate 486, piezoelectric actuator 472, protective front surface 488 and bond pad 452 are formed on top of the interconnect layer 433 as shown in Figure 9(b). The nozzle plate 486 is deposited using a CVD or PVD process.

The formation of a CMOS compatible piezoelectric material within the piezoelectric actuator 472 is of particular interest as this is the key driving element of the actuator. ZnO, AIN and AIN compounds (such as ScAlN) materials can be deposited using low-temperature PVD (e.g. sputtering) processes that do not require post processing such as annealing. These materials also do not require poling.

ZnO, AIN and AIN compounds (e.g. ScAlN) materials are therefore commercially viable materials for the fabrication of a monolithic droplet ejector device. However, the value of *d*₃₁ for these materials is significantly lower than that of PZT. The particular configuration of the nozzle (i.e. the actuatable nozzle plate), which improves ejection efficiency, and the use of two control electrodes, which improves actuation efficiency, counter the lower *d*₃₁ value associated with these materials.

Piezoelectric electrode materials are deposited using a CMOS compatible process such as PVD (including low-temperature sputtering). Typical electrode materials may include titanium (Ti), platinum (Pt), aluminium (Al), tungsten (W) or alloys thereof. The electrodes of the piezoelectric actuator 472 are defined by standard patterning and etch methods.

Protective materials can be deposited and patterned using a spin on and cure method (suitable for polyimides or other polymeric materials). Some materials, such as PTFE, may require more specific deposition and patterning approaches.

Bond pads are deposited using methods such as CVD or PVD (e.g. sputtering).

The liquid chambers and liquid chamber inlets are defined using high aspect ratio Deep Reactive Ion Etching (DRIE) methodologies to arrive at the shape shown in Figure 9(c). The liquid chambers are aligned to the nozzle outlets using a wafer front-back side alignment tool. The wafer may be mounted on a handle wafer during the front-back alignment and etch steps.

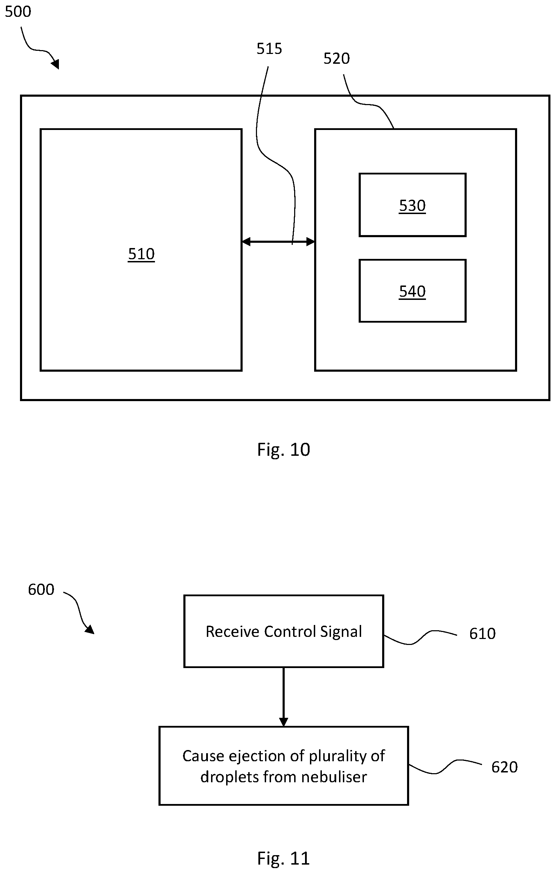

Figure 10 is a schematic illustration of a nebuliser according to an example of the present invention. The nebuliser 500 comprises a plurality of components, including a plurality of droplet ejectors 510, and a controller 520. The controller 520 is configured to exchange signals 515 with the plurality of droplet ejectors 510 to control the plurality of droplet ejectors 510 in accordance with input signals received by the controller 520, for example from a dosing regimen pre-programmed into the nebuliser 500. The controller 520 in this example is realised by one or more processors 530 and a computer-readable memory 540. The memory 540 stores instructions which, when executed by the one or more processors 530, cause the nebuliser 500 to operate as described herein.

Figure 11 is a flowchart illustrating a method of controlling a nebuliser as described herein. The method 600 is a method of controlling a nebuliser having a plurality of droplet ejectors for generating an aerosol from a liquid provided thereto. Specifically, the method 600 comprises receiving 610 a control signal. The control signal may be received from a computer program stored in memory, or may be received as a result of a user input indicative of a demand to start nebulisation, or may be received from an external device. The method 600 further comprises causing 620 ejection of a plurality of droplets of liquid from the nebuliser. The ejection of the plurality of droplets is caused by activation of the plurality of droplet ejectors, for example, by applying the driving waveform described herein (or another driving waveform) to the piezoelectric actuator of a plurality of the plurality of droplet ejectors.

In summary, the present disclosure provides a nebuliser (100) comprising one or more inputs (110), and a plurality of droplet ejectors (126) in fluid communication with the one or more inputs (110). Each droplet ejector comprises a nozzle portion and a piezoelectric actuator. The one or more inputs are for receiving one or more liquids. Each nozzle portion defines a nozzle outlet in fluid communication with the one or more inputs. Each piezoelectric actuator is operable to cause ejection of a liquid received by one of the one or more inputs through the respective nozzle outlet as one or more droplets. The nebuliser is configured to generate an aerosol comprising the respective one or more droplets ejected by one or more of the plurality of droplet ejectors. At least 50%, by mass, of the droplets of the aerosol have a diameter of at least 5 × 10⁻⁶ m.

Throughout the description and claims of this specification, the words "comprise" and "contain" and variations of them mean "including but not limited to", and they are not intended to and do not exclude other components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

## Claims

1. A nebuliser for delivering a substance to the olfactory region, the nebuliser comprising:
one or more inputs for receiving one or more liquids, at least one of the one or more liquids comprising the substance; and
a plurality of droplet ejectors together in fluid communication with the one or more inputs, each droplet ejector comprising:
a nozzle portion defining a nozzle outlet in fluid communication with a one of the one or more inputs; and
a piezoelectric actuator,
wherein each piezoelectric actuator is operable to cause ejection of a liquid received by the one of the one or more inputs through the respective nozzle outlet as one or more droplets,
wherein the nebuliser is configured to generate an aerosol comprising the respective one or more droplets ejected by one or more of the plurality of droplet ejectors, and
wherein at least 50%, by mass, of the droplets of the aerosol have a diameter of at least 5 × 10⁻⁶ m.

2. The nebuliser according to claim 1, wherein at least 90%, by mass, of the plurality of droplets of the aerosol have a diameter of at least 8 × 10⁻⁶ m.

3. The nebuliser of claim 1 or claim 2, wherein the plurality of droplet ejectors comprises a first plurality of droplet ejectors and a second plurality of droplet ejectors, and wherein the nebuliser is configured to:
generate a first aerosol comprising the respective one or more droplets ejected by the first plurality of droplet ejectors; and
generate a second aerosol comprising the respective one or more droplets ejected by the second plurality of droplet ejectors.

4. The nebuliser of any preceding claim, wherein the one or more liquids comprises a first liquid and a second liquid.

5. The nebuliser of claim 4 when dependent on claim 3, wherein the first aerosol is an aerosol of the first liquid and the second aerosol is an aerosol of the second liquid.

6. The nebuliser of claim 4 or claim 5, wherein:
the one or more inputs comprises a first input and a second input;
the first input is for receiving the first liquid;
the second input is for receiving the second liquid;
the first plurality of droplet ejectors is in fluid communication with the first input; and
the second plurality of droplet ejectors is in fluid communication with the second input.

7. The nebuliser of any of claims 3 to 6, wherein the nebuliser is configured to:
commence generation of the first aerosol at a first start time;
cease generation of the first aerosol at a first end time;
commence generation of the second aerosol at a second start time, no earlier than the first start time;
cease generation of the second aerosol at a second end time.

8. The nebuliser of claim 7, wherein the second end time is after the first end time.

9. The nebuliser of any of claims 4 to 8, wherein the second liquid comprises the substance and the first liquid comprises a further substance.

10. The nebuliser of any preceding claim, the nebuliser further comprising the one or more liquids.

11. The nebuliser of any preceding claim, wherein the nebuliser is for delivering the substance to the brain via the olfactory region.

12. The nebuliser of any preceding claim, further comprising a storage portion defining one or more cavities for storing the one or more liquids, wherein the one or more cavities are together in fluid communication with each nozzle outlet and with the one or more inputs, optionally wherein the nebuliser further comprises the one or more liquids within the one or more cavities, and additionally or alternatively wherein the storage portion is removable.

13. The nebuliser according to any preceding claim, further comprising an outlet portion defining a nebuliser outlet through which the aerosol can be output from the nebuliser, optionally wherein the outlet portion is part of the housing.

14. The nebuliser according to claim 13, wherein the outlet portion is in the form of a nose insert.

15. A controller for a nebuliser, the nebuliser for delivering a substance to the olfactory region, the nebuliser comprising:
one or more inputs for receiving one or more liquids, at least one of the one or more liquids comprising the substance; and
a plurality of droplet ejectors together in fluid communication with the one or more inputs, each droplet ejector comprising:
a nozzle portion defining a nozzle outlet in fluid communication with a one of the one or more inputs; and
a piezoelectric actuator,
wherein each piezoelectric actuator is operable to cause ejection of a liquid received by the one of the one or more inputs through the respective nozzle outlet as one or more droplets, and
wherein the controller is configured to:
cause the piezoelectric actuator of each of one or more of the plurality of droplet ejectors to eject one or more droplets to thereby generate an aerosol comprising the one or more droplets ejected by the one or more droplet ejectors, and
wherein at least 50%, by mass, of the droplets of the aerosol have a diameter of at least 5 × 10⁻⁶ m.
